# EUROPÄISCHE PATENTSCHRIFT

(11) **EP 2 607 712 B1**
(45) Veröffentlichungstag und Bekanntmachung des Hinweises auf die Patenterteilung: **13.07.2016**
(21) Anmeldenummer: 11075271.4
(22) Anmeldetag: 22.12.2011
(51) Int. Cl.: F04D 29/02, F04D 29/18, F04D 29/24, F04D 29/52, A61M 1/10, F04D 15/00

(54) **Pumpengehäuse mit einem Innenraum zur Aufnahme eines Pumpenrotors**
Pump housing with an interior for holding a pump rotor
Boîtier de pompe doté d'un espace intérieur destiné à la réception d'un rotor de pompe

(43) Veröffentlichungstag der Anmeldung: 26.06.2013
(73) Patentinhaber: ECP Entwicklungsgesellschaft mbH, 12247 Berlin (DE)
(72) Erfinder: Bredenbreuker, Lars, 10781 Berlin (DE); Schumacher, Jörg, 14513 Teltow (DE)
(74) Vertreter: Pfenning, Meinig & Partner mbB

(56) Entgegenhaltungen:
- EP-A1- 2 047 872
- EP-A1- 2 347 778
- EP-A2- 1 201 932
- WO-A2-03/103745
- DE-C1- 10 059 714
- DE-U1- 29 804 046
- US-B2- 7 393 181

## Beschreibung

Die Erfindung liegt auf dem Gebiet des Maschinenbaus und findet insbesondere in der Medizintechnik Anwendung. Sie bezieht sich auf Pumpen, die mit Pumpenrotoren ausgestattet sind und ein Pumpengehäuse aufweisen, dessen Innenraum den Pumpenrotor aufnimmt. Speziell in der Medizintechnik, jedoch grundsätzlich auch in anderen Anwendungsgebieten der Technik, sind Pumpen bekannt, die radial komprimierbar sind, indem sowohl der Rotor als auch das Pumpengehäuse zur Verringerung des Durchmessers verformbar sind, und die nach dem Transport an den Einsatzort wieder expandierbar sind, um die für eine optimierte Funktion erforderlichen Maße einzustellen. Weiter speziell in der Medizintechnik sind solche Pumpen bekannt, die so weit komprimiert werden können, dass sie über ein Blutgefäß in den Körper eines Patienten einführbar und dort expandierbar sind, um über den Betrieb der Pumpe einen Bluttransport zu unterstützen oder selbständig zu gewährleisten.

Um eine zuverlässige Komprimierbarkeit und Expandierbarkeit zu gewährleisten, sind eine Vielzahl von technischen Aufgaben zu lösen.

Beispielsweise beschreibt hierzu die US-Patentschrift US 7393 181 B2 einen komprimierbaren Pumpenrotor, bei dem Förderschaufeln in Reihen an einer Nabe angeordnet und an diese anklappbar sind.

Aus der US 7841976 B2 ist ein Pumpenrotor bekannt, der in den expandierbaren Teil einer Kanüle einsetzbar ist, um dort nach Expandieren des entsprechenden Kanülenteils betrieben zu werden. Der Rotor wird dazu über eine Welle angetrieben, die durch die Kanüle verläuft. Die so gebildete Pumpe kann durch ein Blutgefäß bis in eine Herzkammer vorgeschoben und dort betrieben werden.

Auch die US 7 927068 B2 beschreibt eine Pumpe mit einem Rotor, der eine Nabe und an- und abklappbare Förderschaufeln aufweist. Die Förderschaufeln werden im Betrieb in Rotation durch den entstehenden Fluidgegendruck in die für den Pumpbetrieb geeignete Position gedrückt.

Aus der DE 100 59 714 C1 ist eine intravasale Pumpe mit einem komprimierbaren und expandierbaren Gehäuse bekannt, das ein Gittergeflecht aufweist. Dieses Gehäuse kann einen Rotor aufnehmen und mittels einer Einführschleuse in ein Blutgefäß eingeführt werden.

Der genannte Stand der Technik setzt sich generell mit den Problemen auseinander, eine Pumpe mit einem Rotor und einem Pumpengehäuse ausreichend radial zu komprimieren, damit sie beispielsweise in ein Blutgefäß eines Patienten einführbar und nach der Einführung expandierbar ist. Später soll die jeweilige Pumpe auch wieder komprimierbar sein, um explantiert werden zu können. Bei dem Kompressions- und Expansionsprozess werden üblicherweise sowohl der Rotor als auch das Gehäuse entsprechend verformt. Eine besondere Herausforderung stellt dabei die Anforderung dar, im Betrieb den Pumpenspalt, d.h. den Zwischenraum zwischen dem Rotor, genauer den radial äußeren Enden der Förderelemente, und der inneren Gehäusewand des Pumpengehäuses zu minimieren, um den Pumpenbetrieb, speziell den Wirkungsgrad und die Kompression der Pumpe, zu optimieren. Der Pumpenspalt muss sehr klein und insbesondere so konstant wie möglich gehalten werden, um das Überströmen an den radial äußeren Enden von Förderelementen eines Rotors zu verhindern, andererseits dürfen die Förderelemente oder andere Elemente des Rotors die Innenwand des Pumpengehäuses möglichst nicht berühren, um bei den hohen üblichen Drehzahlen von mehr als 10000 Umdrehungen pro Minute keine unnötigen Reibungsverluste oder Abrieb zu erzeugen.

Der vorliegenden Erfindung liegt daher vor dem Hintergrund des Standes der Technik die Aufgabe zugrunde, ein Pumpengehäuse sowie eine Pumpe der eingangs genannten Art zu schaffen, das/die eine möglichst genaue und reproduzierbare Einstellung des Pumpenspaltes erlauben.

Die Aufgabe wird gemäß der Erfindung mit den Merkmalen des Patentanspruchs 1 gelöst.

Das Pumpengehäuse weist demgemäß eine Gehäusehaut sowie wenigstens ein Verstärkungselement auf, wobei im voll expandierten Zustand des Gehäuses die Gehäusehaut im Umfangsrichtung durch die Expansion des Verstärkungselementes gespannt ist und wobei wenigstens ein in Umfangsrichtung umlaufendes dehnfestes Element vorgesehen ist, das im expandierten Zustand gegenüber dem kraftfreien Zustand in Umfangsrichtung weniger als 5%, insbesondere weniger als 1%, gedehnt ist und das eine weitere Expansion des Pumpengehäuses begrenzt.

Die Gehäusehaut kann aus einem leicht verformbaren flexiblen, insbesondere biegeschlaffen Kunststoff bestehen und beispielsweise ein Polymer enthalten. Die Gehäusehaut kann einfach um das Verstärkungselement herum angeordnet oder mit diesem durch übliche Fügetechniken wie Kleben oder Schweißen verbunden sein. Das Verstärkungselement kann auch durch Eingießen in die Gehäusehaut integriert sein.

Üblicherweise ist das Verstärkungselement derart aufgebaut und angeordnet, dass es die Gehäusehaut radial von der Innenseite aus stützt. Dies ist auch bei einer Integration, beispielsweise einem Einguss, des Verstärkungselementes in die Gehäusehaut möglich.

Bei einer Expansion des Pumpengehäuses von einem komprimierten Zustand aus verformt sich das Verstärkungselement oder eine Gruppe von Verstärkungselementen und spannt die Gehäusehaut, indem es auf diese eine radial nach außen wirkende Kraft ausübt. Die hydrodynamischen und insbesondere hämodynamischen Eigenschaften des Pumpengehäuses sind insbesondere dann verbessert, wenn die Gehäusehaut gespannt, insbesondere faltenfrei gespannt ist und die Innenwand des Pumpengehäuses, die den Innenraum zur Aufnahme des Pumpenrotors begrenzt, möglichst glatt ist.

Jedoch sollte dies nicht dazu führen, dass durch die auf die Gehäusehaut wirkenden Kräfte je nach der Größe der durch die Verstärkungselemente wirkenden Kräfte variable Dehnungskräfte erzeugt werden, so dass die Maße des Innenraums des Gehäuses variieren. Zu diesem Zweck sieht die Erfindung ein in Umfangsrichtung umlaufendes dehnfestes Element vor, das durch die radial nach außen wirkenden Kräfte des Verstärkungselements / der Verstärkungselemente nicht wesentlich dehnbar ist. Das dehnfeste Element ist vorteilhaft in dem axialen Abschnitt des Pumpengehäuses angeordnet, der den Pumpenrotor aufnimmt. Das dehnfeste Element kann dabei auch die gesamte axiale Länge des Pumpenrotors abdecken.

Auch das dehnfeste Element kann eine biegeschlaffe Folie sein, die beispielsweise wesentlich dehnfester ist als die Gehäusehaut und diese radial umgeben kann.

Das dehnfeste Element kann auch lediglich die Verstärkungselemente umgeben, selbst von der Gehäusehaut radial umgeben sein oder die Gehäusehaut ersetzen.

Das Pumpengehäuse weist vorteilhaft zumindest einen axialen Abschnitt auf, in dem der Innenraum im Wesentlichen zylindrisch geformt ist. Dieser zylindrisch geformte axiale Innenraumbereich kann beispielsweise den Pumpenrotor aufnehmen.

Eine solche zylindrische Form soll auch annähernd kreiszylindrische Formen umfassen, die als polygonale Prismen oder als Polyeder gebildet sind, die jeweils einem Kreiszylinder einbeschrieben sind und durch die Stützung der Gehäusehaut durch Streben entstehen. Die einzelnen Begrenzungsflächen, aus denen die Form besteht, können z.B. Rauten sein.

Das Pumpengehäuse bzw. sein Innenraum kann axial mehr oder weniger weit über die Länge des Pumpenrotors respektive den axialen Abschnitt, der für die Aufnahme des Pumpenrotors vorgesehen ist, hinausreichen, beispielsweise wenigstens 2 cm, insbesondere wenigstens 5 cm, weiter insbesondere wenigstens 8 cm. Der über die Länge des zur Aufnahme des Pumpenrotors vorgesehenen axialen Abschnitts hinausgehende Bereich des Pumpengehäuses kann vom Pumpenrotor aus gesehen distal liegen, d.h. zu dem Ende des Pumpengehäuses hin, an dem dieses seine Einsaugseite mit einer Einsaugöffnung aufweist. Am proximalen Ende des Pumpengehäuses ist eine Ausstoßöffnung für das zu fördernde Fluid vorgesehen. Es kann aber auch der Rotor, zumindest teilweise, über die durch eine Gehäusehaut umschlossene Struktur herausragen.

Die Einsaugöffnung des Pumpengehäuses kann beispielsweise eine stirnseitige Öffnung sein, die vorteilhaft von einem Einsaugkäfig abgedeckt ist. Der Einsaugkäfig kann beispielsweise die Form einer Kugelkalotte aufweisen oder in Ballonform auch zunächst eine Erweiterung über den Durchmesser des Pumpengehäuses hinaus vorsehen, die zum distalen Ende hin durch eine Kalotte oder auch eine mit einem Pigtail versehene Spitze abgeschlossen ist. Der Ansaugkäfig kann durch fortgesetzte Verstärkungselemente des Pumpengehäuses gebildet sein.

Das Pumpengehäuse kann bis zu seinem Einsaugende zylindrisch geformt sein und vorteilhaft am einsaugseitigen Ende eine trichterförmige Erweiterung aufweisen, aus der heraus die Verstärkungselemente austreten und einen ballonartig erweiterten Ansaugkäfig bilden.

An der Ausstoßseite bzw. am ausstoßseitigen Ende weist das Pumpengehäuse vorteilhaft ebenfalls eine stirnseitige Öffnung auf. Dort können ebenfalls Verstärkungselemente aus der Wand des Pumpengehäuses austreten und zur zentralen Symmetrieachse des Pumpengehäuses führen, wo sie einen Katheter halten und zentrieren, der koaxial zum Pumpengehäuse mit geringerem Durchmesser als das Pumpengehäuse verläuft und beispielsweise eine Antriebswelle für einen Pumpenrotor aufnehmen kann.

Die Form und Größe sowie die Vorbehandlung des Verstärkungselements / der Verstärkungselemente sind vorteilhaft derart gewählt, dass die elastischen, über die tatsächliche radiale Expansion des Gehäuses hinausgehenden Kräfte des Verstärkungselements / der Verstärkungselemente im expandierten Zustand des Gehäuses so groß sind, dass die radial nach außen gerichteten Überschusskräfte größer sind als 1 N, vorzugsweise 4 N. Dies bedeutet, dass auch eine um die Überschusskräfte verminderte radiale Kraft der Verstärkungselemente noch zu einer vollen Expansion des Gehäuses führen würde.

Damit ist sichergestellt, dass das Gehäuse auch durch einen bestimmten begrenzten radialen Druck von außen (maximal von derselben Größe wie die Überschusskräfte) nicht verformbar, insbesondere nicht bezüglich des Durchmessers des Innenraums komprimierbar ist. Zu einer Kompression des Pumpengehäuses sind Kräfte nötig, die zumindest die radial von innen wirkenden Kräfte der Verstärkungselemente übersteigen.

Damit ist insbesondere bei einer Lage des Pumpengehäuses in der Aortenklappe sichergestellt, dass beim Schließen der Klappe das Pumpengehäuse nicht komprimiert und dadurch der Rotor eingeklemmt oder durch Berührung am Pumpengehäuse abgebremst wird. Ein solcher Kontakt würde einerseits zum Verlust der Pumpleistung, andererseits auch zur Erzeugung von Abrieb und zur Hämolyse, d.h. zur Beschädigung von Blut durch wirkende Scher- und Mahlkräfte, führen.

Das Verstärkungselement / die Verstärkungselemente kann/können beispielsweise elastisch komprimierbare Streben aus einem Metall oder einem Kunststoff sein, die zur Expansion des Pumpengehäuses freigegeben werden und sich elastisch verformen. Ein Verstärkungselement kann beispielsweise auch als ringförmig umlaufendes Element, beispielsweise als offener Kreisring, gebildet sein, der am Umfang des Pumpengehäuses umläuft und als ganzer zwischen einem spiralig aufgewickelten komprimierten Zustand und einem expandierten Zustand verformbar ist, in dem er zu einem Kreisring oder einer Wicklung vergrößerten Durchmessers expandiert ist.

Es ist auch denkbar, dass das Verstärkungselement / die Verstärkungselemente ein Gerüst oder Gewölbe bilden, das durch Schwenken einzelner Elemente gegeneinander expandiert. Bei der Bildung eines solchen Gerüsts oder eines Gewölbes kann auch vorgesehen sein, dass diese im expandierten Zustand in einer selbststabilisierten Position einrasten.

Eine vorteilhafte Ausgestaltung der Erfindung sieht vor, dass ein durch das Verstärkungselement / die Verstärkungselemente im expandierten Zustand des Gehäuses gebildetes Gerüst oder Gewölbe radial nach innen gerichteten Kräften von mindestens 1 N, vorzugsweise 4 N, widersteht, ohne einzufallen.

Wenn die radial nach innen gerichteten Kräfte dieses Maß deutlich übersteigen, gibt das Gewölbe bzw. das Gerüst nach, so dass das Pumpengehäuse seine Außenmaße durch Kompression reduzieren und nach Wegfall der nach innen gerichteten Kräfte wieder expandieren kann. Das Zusammenbrechen des Gewölbes oder Gerüsts kann reversibel oder irreversibel sein.

Eine weitere vorteilhafte Ausgestaltung der Erfindung sieht vor, dass das dehnfeste Element durch eine biegeschlaffe Folie, insbesondere durch die Gehäusehaut, gebildet ist.

Es kann auch vorgesehen sein, dass das dehnfeste Element durch einen in Umfangsrichtung des Gehäuses umlaufenden, wenigstens abschnittsweise das Verstärkungselement umgebenden Ring gebildet ist. Insbesondere kann das dehnfeste Element das Verstärkungselement / die Verstärkungselemente in dem Bereich umgeben, in dem der Pumpenrotor aufgenommen wird.

Vorteilhaft kann zudem vorgesehen sein, dass das dehnfeste Element an der radial äußeren Seite der Gehäusehaut angeordnet ist.

Zur Stabilisierung des dehnfesten Elementes, insbesondere auch der gesamten Gehäusehaut, kann vorteilhaft vorgesehen sein, dass das dehnfeste Element in Umfangsrichtung verlaufende dehnfeste Fasern, insbesondere Glasfasern oder Kohlenstofffasern, aufweist. Auch andere Fasern oder Verstärkungselemente sind zur Verbesserung der Dehnfestigkeit denkbar.

Eine besondere Ausführungsform der Verstärkungselemente sieht vor, dass diese ein flächenartiges, zweidimensionales Gitter bilden, das in die Form einer Röhre gebogen ist. Die einzelnen Gitterelemente sind vorteilhaft leicht gegeneinander verschiebbar und/oder schwenkbar oder auch elastisch verformbar bzw. biegbar, so dass eine Verformung des röhrenförmigen Gebildes einfach möglich ist. Die Verstärkungselemente können beispielsweise sägezahnförmig oder mäanderförmig in Umfangsrichtung des Pumpengehäuses umlaufen.

Es kann auch vorteilhaft vorgesehen sein, dass mehrere gegeneinander schwenkbare Verstärkungselemente in einem ersten Schwenkzustand gemeinsam die Form einer Röhre bilden und in einem zweiten Schwenkzustand radial gegenüber der Röhrenform komprimiert sind. Zu diesem Zweck können die Verstärkungselemente beispielsweise an einzelnen Punkten miteinander verbunden sein und Gelenke bilden, wobei die Gelenkfunktion durch scharnierartige Geräte, jedoch auch durch elastische Verformung realisiert werden kann.

Es kann auch vorteilhaft vorgesehen sein, dass der Gehäuseinnenraum in Axialrichtung sich verjüngend, insbesondere konisch, zuläuft. Dies eröffnet die Möglichkeit, den einzubringenden Rotor im Gehäuseinnenraum axial so weit zu verschieben, dass der Gehäusespalt, d.h. der Zwischenraum zwischen der Innenwand des Gehäuseinnenraums und den radial äußersten Enden des Rotors, insbesondere der Förderelemente des Rotors, optimiert, beispielsweise minimiert ist. Der Konuswinkel des Gehäuseinnenraums kann dabei vorteilhaft zwischen 0,5° und 5°, insbesondere zwischen 0,5° und 2° betragen.

Vorteilhaft kann auch die Außenkontur des Pumpenrotors in demselben Sinn konisch zulaufen wie der Gehäuseinnenraum, weiter vorteilhaft mit im Wesentlichen demselben Konuswinkel. Beim Betrieb des Rotors kann dann dieser axial in dem Gehäuseinnenraum so weit verschoben werden, bis die optimale Pumpleistung erreicht ist. Dies kann beispielsweise durch eine Durchflussmessung und/oder in Verbindung mit einer Leistungsmessung des Antriebsmotors des Pumpenrotors ermittelt werden. Beispielsweise kann der Rotor so weit bewegt werden, bis eine deutliche Abbremsung eintritt, die für eine Kontaktaufnahme des Rotors mit der Innenwand des Gehäuseinnenraums spricht, und darauf kann der Rotor um einen definierten axialen Abstand wieder zurückbewegt und in dieser axialen Position fixiert werden.

Der Gehäuseinnenraum kann aber auch eine leicht konvexe (ballige) oder auch konkave Form aufweisen. Dies ist insbesondere dann vorteilhaft, wenn der Rotor durch die Verformung im Strömungsfeld bei unterschiedlichen Betriebszuständen (z.B. Drehzahlen) unterschiedliche Außenkonturen aufweist. Je nach Grundform des Rotors ist dann eine Gehäuseform erforderlich, die den jeweiligen Erfordernissen aller Betriebszustände gerecht wird.

Unabhängig davon, ob ein konischer Rotor oder ein in seiner Kontur zylindrisch gestalteter Rotor und ein Gehäuseinnenraum in konisch zulaufender oder streng zylindrischer Form verwendet wird, sollte der Pumpenspalt zwischen 0,01 mm und 1 mm, insbesondere zwischen 0,01 mm und 0,3 mm, weiter vorteilhaft zwischen 0,03 mm und 0,15 mm betragen. Dabei ist als Pumpenspalt der minimale Abstand zwischen einem radial äußersten Ende eines Teils des Rotors und der Innenwand des Gehäuseinnenraums bezeichnet, der bei der Rotation des Rotors auftritt. In jedem Fall sollte vorteilhaft sichergestellt werden, dass der Rotor im normalen Betrieb das Pumpengehäuse nicht berührt.

Um eine optimierte Komprimierbarkeit und Expandierbarkeit des Pumpengehäuses zu erzielen, kann vorteilhaft vorgesehen sein, dass das Verstärkungselement / die Verstärkungselemente aus einem superelastischen Werkstoff, insbesondere einer superelastischen Legierung, weiter insbesondere Nitinol, besteht/bestehen. Derartige Werkstoffe sind bedenkenlos sehr stark komprimierbar und können auch als Gedächtniswerkstoffe, insbesondere Gedächtnislegierungen, ausgeführt sein, die unter bestimmten physikalischen Bedingungen, beispielsweise bei Eintreten eines bestimmten Temperaturwechsels oder einer Zieltemperatur, eine vorher gewählte Form einnehmen.

Die Erfindung bezieht sich außer auf ein Pumpengehäuse der oben beschriebenen Art auch auf eine Blutpumpe mit einem entsprechenden Pumpengehäuse und mit einem Rotor.

Der Rotor kann beispielsweise aus einem Kunststoff, insbesondere einem Polyurethan, und vorteilhaft auch aus einem Schaumstoff bestehen. Er weist ein oder mehrere Förderelemente auf, die in radialer und/oder axialer Richtung bezüglich der Rotationsachse eine Flüssigkeit fördern. Die Förderelemente können als Schaufelblätter oder als ein einziges durchlaufendes Schaufelblatt ausgebildet sein, wobei das einzelne Schaufelblatt oder eine Serie von Schaufelblättern helixförmig um eine zentrale Nabe umläuft.

Es können auch zwei schraubenförmig umlaufende Förderschaufeln vorgesehen sein. Die Förderschaufeln können freitragend ohne eine Nabe ausgebildet sein, oder sie können an einer zentralen Nabe befestigt oder integral mit dieser hergestellt sein. Vorteilhaft können die Nabe und die Förderelemente aus demselben Material bestehen.

Die Förderelemente können so ausgestaltet sein, dass sie zur Kompression an die Nabe angeklappt und zur Expansion abgeklappt werden können. Insbesondere nehmen die Förderelemente im kraftfreien und ruhenden Zustand des Rotors eine Mittelposition ein, die weder der komprimierten Position noch der expandierten Position entspricht. Zur Einnahme der komprimierten Position wird ein radialer Druck von außen auf die Förderelemente ausgeübt, um diese eng an eine Nabe anzulegen. Der radiale Druck kann beispielsweise durch Kompression des den Rotor umgebenden Gehäuses aufgebracht werden.

Wird der radiale Druck reduziert, so richten sich die Förderelemente in Radialrichtung ein Stück weit auf. Eine weitere Aufrichtung kann durch den Fluidgegendruck zu Beginn der Rotation des Rotors in dem zu fördernden Fluid bewirkt werden.

Die Förderschaufeln sind typisch derart gestaltet, dass sie im Betrieb bei einer optimierten Drehzahl, insbesondere bei der Maximaldrehzahl der Pumpe, maximal in Radialrichtung aufgerichtet sind und somit die größtmögliche Förderwirkung auf das Fluid ausüben. In diesem Zustand der maximalen Aufrichtung müssen die Maße des Innenraums des Pumpengehäuses und des Rotors derart aufeinander abgestimmt sein, dass keine Berührung zwischen beiden stattfindet.

Es kann auch vorgesehen sein, dass durch den Fluidgegendruck die einzelnen Förderelemente über den Zustand maximaler radialer Aufrichtung hinaus verformt werden, derart, dass sie durch den Fluiddruck ein Stück weit wieder radial komprimiert werden.

Sind entlang einer Nabe eine Vielzahl von einzelnen Förderelementen vorgesehen, so können diese vorteilhaft derart angeordnet und ausgerichtet sein, dass sie insgesamt die Kontur einer helixförmigen Förderschaufel bilden. Die einzelnen Förderelemente sind dann jeweils einzeln an die Nabe anklappbar und expandierbar.

Der Rotor, insbesondere die Nabe, vorteilhaft jedoch sowohl die Nabe als auch die Förderelemente, besteht vorteilhaft aus einem Polyurethan, beispielsweise auch aus einem geschäumten Polyurethan, einem thermoplastischen Elastomer, einem Gummi oder einem superelastischen Material, insbesondere einem superelastischen Polymer.

Die Erfindung bezieht sich außer auf ein Pumpengehäuse und eine Blutpumpe mit einem solchen Pumpengehäuse und einem Rotor auch auf eine Blutpumpeneinrichtung mit einem Pumpengehäuse, einem Rotor und einem sich an einem Ende des Pumpengehäuse anschließenden Katheter. Der Katheter kann beispielsweise Teil einer Manipuliereinrichtung sein, die erlaubt, das Pumpengehäuse und den Rotor durch ein Blutgefäß eines Patienten bis in eine Herzkammer wenigstens teilweise einzuschieben.

Der Katheter weist insbesondere ein längs durchlaufendes Lumen auf, in dem eine Antriebswelle für den Rotor der Pumpe angeordnet ist. Es kann aber auch zum Antrieb des Rotors ein Motor in unmittelbarer Nähe des Pumpengehäuses angeordnet sein.

Der Katheter kann vorteilhaft direkt mit dem Pumpengehäuse zusammenhängen, wobei entweder am Ende des Pumpengehäuses und/oder zum Beginn oder im Verlauf des Katheters radiale Öffnungen für das zu fördernde Fluid vorgesehen sein können. Der Katheter weist vorteilhaft einen wesentlich geringeren Durchmesser auf als der Innenraum des Pumpengehäuses und ist am Ende des Pumpengehäuses befestigt. Er ist vorteilhaft koaxial und konzentrisch mit dem Pumpengehäuse verbunden. Die Verbindung kann beispielsweise mittels der Verstärkungselemente des Pumpengehäuses gestaltet sein, die über das Pumpengehäuse axial hinausragen und zentrisch radial nach innen abgebogen sein können, um dort mit dem Katheter verbunden zu sein. Somit können die Fortsätze der Verstärkungselemente/Streben des Pumpengehäuses den Katheter halten und zentrieren.

Im Folgenden wird die Erfindung anhand eines Ausführungsbeispiels in einer Zeichnung gezeigt und anschließend beschrieben. Dabei zeigt
- Fig. 1: eine schematische Darstellung einer über ein Blutgefäß in eine Herzkammer eingeführten Blutpumpe,
- Fig. 2: eine Blutpumpe in einer Herzkammer im Längsschnitt,
- Fig. 3: eine Blutpumpe in einer Seitenansicht,
- Fig. 4: einen Teil eines Pumpengehäuses mit Verstärkungselementen,
- Fig. 5: eine Seitenansicht eines Pumpengehäuses mit Verstärkungselementen,
- Fig. 6: Verstärkungselemente für ein Pumpengehäuse in expandierter Form,
- Fig. 7: die Verstärkungselemente aus Fig. 6 in komprimierter Form,
- Fig. 8: einen Längsschnitt durch einen Teil eines Pumpengehäuses mit einem Rotor, wobei der Pumpeninnenraum konisch gestaltet ist,
- Fig. 9: einen Teil eines Längsschnittes eines Pumpengehäuses mit einem Rotor, wobei der Gehäuseinnenraum zylindrisch ausgebildet ist,
- Fig. 10: einen schematischen Längsschnitt durch eine Blutpumpe,
- Fig. 11: einen Längsschnitt durch einen Teil eines Pumpengehäuses mit einem Verstärkungselement,
- Fig. 12: einen Längsschnitt durch ein Pumpengehäuse mit kreisringförmigen Verstärkungselementen,
- Fig. 13: einen Längsschnitt durch einen Teil einer Blutpumpe mit einem Pumpengehäuse sowie einem dehnfesten Element und Verstärkungselementen,
- Fig. 14: einen Längsschnitt durch einen Teil einer Blutpumpe mit einem Ventil stromabwärts des Rotors,
- Fig. 15: eine ähnlich Anordnung wie in Fig. 14 mit einem Ventil stromaufwärts des Rotors,
- Fig. 16: eine Anordnung, bei der ein Rotor über ein Ventil ein Fluid durch einen Käfig ausstößt,
- Fig. 17: eine Ausgestaltung eines Ventils in zwei Stellungen sowie
- Fig. 18: eine weitere Ausgestaltung eines Ventils in zwei Stellungen.

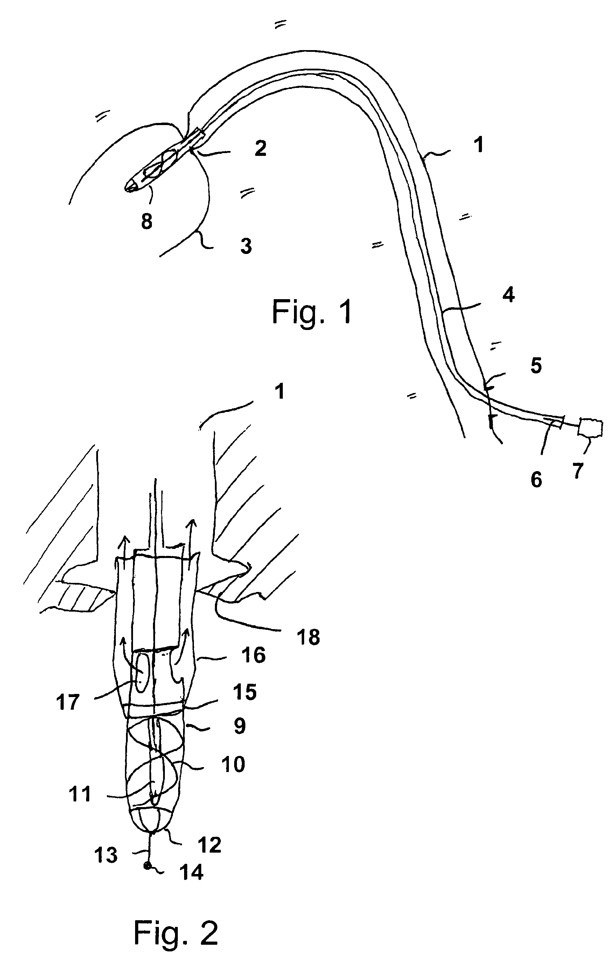

**Figur 1** zeigt schematisch ein Blutgefäß 1 eines menschlichen Körpers, das über eine Herzklappe 2 mit einer Herzkammer 3 verbunden ist und in das ein Katheter 4 über eine Schleuse 5 eingeführt ist. Der Katheter 4 weist einen Kanal (Lumen) in seinem Inneren auf, über den eine Antriebswelle 6 von einem äußeren Antriebsmotor 7 bis in eine in die Herzkammer 3 eingeführte Herzpumpe 8 führt. Die Herzpumpe 8 kann beispielsweise in der bekannten Seldingertechnik in das Blutgefäß eingeführt und durch dieses bis zur Herzkammer vorgeschoben werden.

Die Herzpumpe 8 weist in ihrem Inneren einen Rotor auf, der mittels der Antriebswelle 6 mit einigen tausend, typischerweise zwischen 10000 und 50000 Umdrehungen pro Minute antreibbar ist und in axialer Richtung Blut fördert. Der Rotor ist von einem Pumpengehäuse umgeben, das eine distale Ansaugöffnung aufweist, über die das Blut in der Herzkammer 3 angesaugt werden kann.

Derartige Blutpumpen werden zum Ersatz oder zur Unterstützung der natürlichen Herzfunktion entweder vorübergehend oder auch dauerhaft eingesetzt. Gerade beim unterstützenden Einsatz einer solchen Herzpumpe ist es vorteilhaft, wenn die natürliche Tätigkeit des Herzens unbeeinflusst bleibt, so dass auch das Herz selbst zur Pumpfunktion durch die Herzklappe beiträgt. Das Herz kann hierzu entweder durch die Pumpe hindurch unterstützend Pumparbeit leisten oder an dieser vorbei durch die Herzklappe parallel zur Herzpumpe Blut fördern.

In **Figur 2** ist eine Ausführungsform einer Herzpumpe mit einem annähernd zylindrischen Pumpengehäuse 9 in expandierter Form gezeigt, in dem sich ein Rotor 10 befindet. Der Rotor 10 weist beispielsweise ein wendelförmig an einer Nabe 11 umlaufendes Förderelement in Form einer Förderschaufel auf. Der Raum, den der Rotor bei seiner Rotation einnimmt, ist zylindrisch und an den Gehäuseinnenraum des Gehäuses 9 möglichst genau angepasst.

Das Pumpengehäuse 9 weist an seinem distalen Ende einen Ansaugkäfig 12 auf, der von einigen Streben gebildet ist, die gleichzeitig Verstärkungselemente des Pumpengehäuses 9 bilden, in das Material des Pumpengehäuses eingebettet sind und über dieses axial in distaler Richtung hinausragen.

Am distalen Ende des Ansaugkäfigs 12 ist eine atraumatische Spitze 13 angeordnet, die im Beispiel die Form einer Kugel 14 aufweist, welche dafür sorgt, dass die Pumpe beim Einschieben in das Blutgefäß und die Herzkammer keine Gefäßwände oder Herzwände beschädigt und dass sich das Ansaug-Ende mit der Ansaugöffnung 12 des Pumpengehäuses 9 bei der Förderung von Blut nicht an einer Gefäßwand festsaugt.

In einem axialen Bereich 15 des Pumpengehäuses ist ein folienartiger Abströmmantel 16 mit dem Pumpengehäuse fluiddicht verbunden. Der Abströmmantel 16 besteht aus einer flexiblen, biegeschlaffen, sehr dünnen Folie, die die Abströmöffnungen 17 des Pumpengehäuses 9, die mantelseitig angeordnet sind, überdeckt und über diese hinaus ein Stück weiter in proximaler Richtung der Pumpe, d.h. in Richtung der Schleuse 5, reicht. Die Herzklappe, schematisch angedeutet durch das Bezugszeichen 18, drückt den Abströmmantel 16 an die Fortsetzung des Pumpengehäuses 9 und schließt somit die Herzkammer gegenüber dem Blutgefäß 1. Wird die Pumpe betrieben, so erzeugt sie einen Überdruck und stößt aus den Abströmöffnungen 17 Blut in radialer und axialer Richtung aus, was dazu führt, dass der Abströmmantel 16 sich radial hebt und dass bei ausreichendem Druck die Herzklappe 18 geöffnet wird, um durch den Abströmmantel 16 an der proximalen Fortsetzung des Pumpengehäuses 9 vorbei Blut in das Blutgefäß 1 strömen zu lassen. Dies ist insbesondere in der Phase der Fall, wenn die Restfunktion des Herzens, das durch die Pumpe unterstützt wird, einen zusätzlichen Druckanstieg im Einströmbereich der Pumpe bewirkt.

Hierdurch ist sichergestellt, dass der Blutstrom von der Herzkammer in das Blutgefäß mit der zeitlichen Struktur der natürlichen Herztätigkeit moduliert ist.

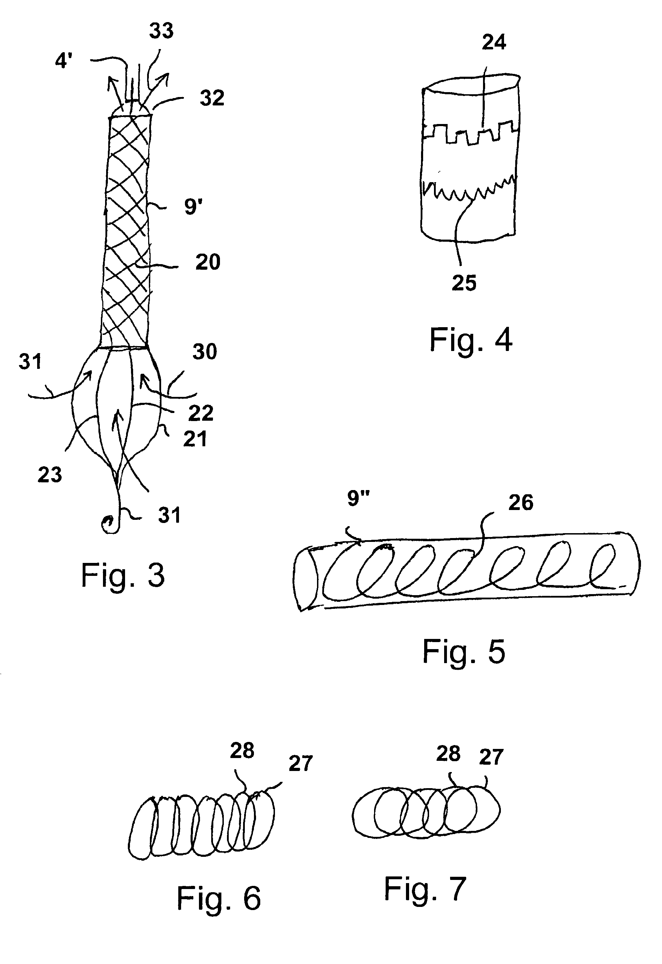

**Figur 3** zeigt in einer Seitenansicht eine weitere Blutpumpe mit einem Pumpengehäuse 9', bei dem gitterartige Verstärkungselemente 20 in Form von drahtartigen Streben dargestellt sind. Die Verstärkungselemente 20 sind am distalen Ende des Pumpengehäuses 9' in freien Streben 21, 22, 23 zu einem Ansaugkäfig fortgesetzt, der das Einströmen von Blut, angedeutet durch die Pfeile 30, 31, erlaubt.

Der Ansaugkäfig 21, 22, 23 weist zudem ein sogenanntes Pigtail 31 an seinem distalen Ende auf, das das Festsaugen des Ansaugkäfigs an einer Gefäßwand verhindern soll.

Am proximalen Ende weist das Pumpengehäuse 9' stirnseitig eine Ausstoßöffnung 32 auf, aus der das Blut, angedeutet durch den Pfeil 33, in ein Blutgefäß ausgestoßen werden kann.

Die proximale Fortsetzung des Pumpengehäuses 9' ist durch einen Katheter 4' gebildet, der in seinem Inneren einen Hohlraum zur Aufnahme einer Antriebswelle für die Pumpe aufweist.

Das Pumpengehäuse 9' ist mit den Verstärkungselementen 20 derart gebaut, dass es mitsamt dem Ansaugkäfig radial gut komprimierbar ist.

Die Verstärkungselemente 20 können beispielsweise in eine biegeschlaffe Folie integriert sein, die die Gehäusehaut bildet und nicht dehnbar ist, so dass sie nach der Expansion der Streben 20 eine Expansion des Pumpengehäuses 9' über einen fest definierten Zustand hinaus verhindert.

**Figur 4** zeigt in einer Seitenansicht einen zylindrischen Abschnitt eines Pumpengehäuses mit eingelegten Verstärkungselementen 24, 25, die in Umfangsrichtung umlaufend, im Fall des Verstärkungselementes 24 mäanderförmig und im Fall des Verstärkungselementes 25 in Sägezahnform gestaltet sind. Diese Form erlaubt eine einfache radiale Expansion und Kompression des Gehäuses. Es können zusätzlich quer dazu verlaufende Verstärkungselemente vorgesehen sein.

In **Figur 5** ist schematisch ein Verstärkungselement 26 angedeutet, das als Schraubenfeder in ein Gehäuse 9'' integriert ist. Auch dieses Verstärkungselement 26 ist in einfacher Form komprimierbar.

**Figur 6** zeigt eine Vielzahl von Kreisringen 27, 28, die insgesamt zu einer Röhre geformt sind und in dieser Weise ein nicht dargestelltes Pumpengehäuse stützen können.

Die Kreisringe können gegeneinander um eine in der Zeichnungsebene liegende Achse gedreht werden, so dass sie alle in derselben Ebene liegen, wie in **Figur 7** dargestellt. In dieser Position sind die Verstärkungselemente 27, 28 in einer Radialrichtung (senkrecht zur Zeichnungsebene) sehr stark komprimiert, während sie in der darauf senkrecht stehenden Radialrichtung unveränderte Maße aufweisen.

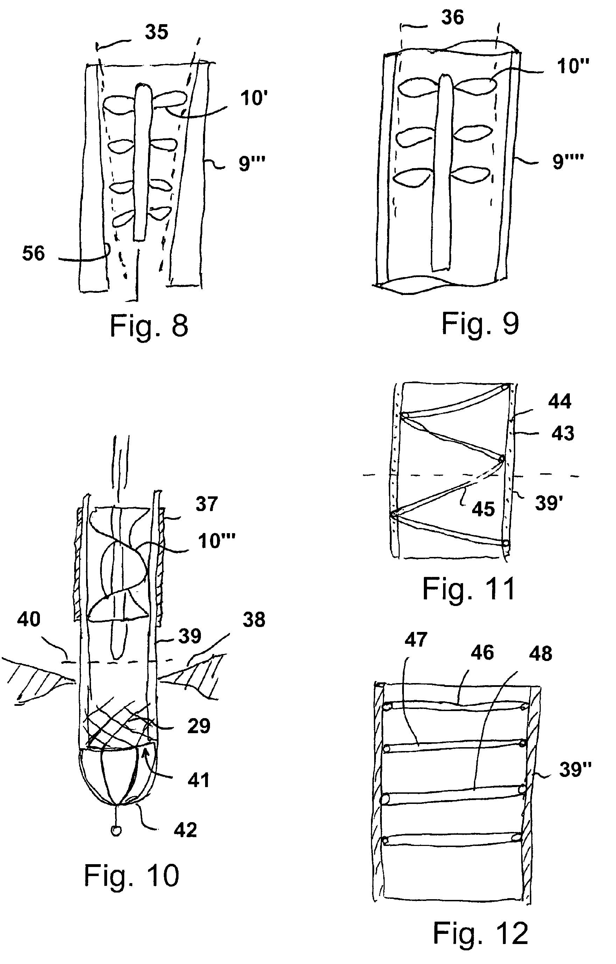

In **Figur 8** ist ein Längsschnitt eines Teils eines Pumpengehäuses mit einem Rotor dargestellt, wobei das Pumpengehäuse 9''' einen konisch in Richtung des Pfeils 34 spitz zulaufenden Gehäuseinnenraum aufweist. Der Konuswinkel ist in der Figur zur besseren Erkennbarkeit übertrieben. Geeignet sind Konuswinkel in der Größenordnung weniger Grad, insbesondere zwischen 0,5° und 5°, weiter insbesondere kleiner als 2°. Die gestrichelt dargestellte Außenkontur 35 des Rotors 10' ist ebenfalls konisch ausgebildet, und zwar vorteilhaft mit demselben Konuswinkel wie der Innenraum des Gehäuses 9'''.

Wird der Rotor 10', beispielsweise mithilfe der nicht dargestellten Antriebswelle, in den enger werdenden Bereich des Konus des Pumpengehäuses 9''' hineingezogen, so ergibt sich eine immer engere Passung zwischen der Außenkontur des Rotors 10' und der Innenwand des Gehäuses 9'''. Der Rotor kann so weit gezogen werden, bis der optimale Pumpenspalt erreicht ist.

Im Gegensatz zu Fig. 8 zeigt **Figur 9** ein ideal zylindrisches Pumpengehäuse 9'''' mit einem ebenso zylindrischen Innenraum, in dem ein Rotor 10'' angeordnet ist, der ebenfalls eine zylindrische Kontur, angedeutet durch die gestrichelte Linie 36, aufweist. Diese Konfiguration ist unempfindlich gegen axiale Verschiebungen des Rotors 10'' gegenüber dem Gehäuse 9''''.

Eine solche Konstellation kann auch unabhängig von der Idee des Hauptanspruchs, nämlich der Verwendung eines dehnfesten Elements zur Begrenzung der radialen Expansion eines Pumpengehäuses, allgemein bei Rotorpumpen eingesetzt werden.

**Figur 10** zeigt schematisch den Längsschnitt eines Pumpengehäuses 39 mit einem Rotor 10'''. Der Rotor 10''' ist in seiner Außenkontur zylindrisch ausgebildet und befindet sich in einem zylindrischen Abschnitt des Gehäuses 39. In die Gehäusewand des Gehäuses 39 sind Verstärkungselemente 29 durch Eingießen integriert, die für die Expansion der Pumpe zuständig sind und die Gehäusehaut spannen. Es ist ein dehnfestes Element 37 in Form eines Ringstreifens dargestellt, der die Gehäusehaut des Gehäuses 39 radial umgibt und somit die radiale Expansion des Pumpengehäuses 39 effektiv begrenzt. Die Verstärkungselemente 29 sind auch im expandierten Zustand der Pumpe noch so weit gespannt, dass sie auf eine weitere Expansion des Pumpengehäuses 39 mit einer gewissen Überschusskraft hinwirken. Dies führt dazu, dass eine Kraft, die radial von außen auf das Pumpengehäuse 39 wirkt und eine Größe zwischen 1 und 25 N nicht übersteigt, nicht zu einer radialen Kompression des Gehäuses 39 führt.

Das dehnfeste Element 37 kann beispielsweise als hochfeste Kunststofffolie, insbesondere auch mit in Umfangsrichtung umlaufenden Verstärkungsfasern, beispielsweise aus oder mit Glasfaser- oder Kohlenfaserwerkstoffen oder auch aus oder mit Aramidfasern oder Nylonfasern, ausgestaltet sein.

Die Herzklappe eines Herzens, in das die Pumpe eingeführt ist, ist in Fig. 10 mit dem Bezugszeichen 38 angedeutet. Es ist auch dargestellt, dass das Pumpengehäuse 39 axial in distaler Richtung über das Ende 40 des Rotors 10''', dargestellt durch eine gestrichelte Linie, hinausragt. Die Pumpe ist derart in einer Herzkammer positioniert, dass der Rotor 10''' außerhalb der Herzkammer in einem Blutgefäß liegt, während ein Teil des Gehäuses 39 distal von der gestrichelten Linie 40 in die Herzkammer hineinragt. In der Herzkammer selbst ist die Ansaugöffnung 41 angeordnet, die durch einen Ansaugkäfig 42 überdeckt ist.

Die Länge der Gehäusefortsetzung des Gehäuses 39 distal vom Ende 40 des Rotors bis zur Ansaugöffnung 41 kann zwischen wenigen Millimetern und einigen Zentimetern, beispielsweise zwischen 0,5 und 10 cm, insbesondere zwischen 0,5 und 5 cm oder 0,5 und 2 cm betragen.

**Figur 11** stellt eine andere Struktur des Pumpengehäuses 39' dar, bei der eine Gehäuseaußenhaut 43 mit in Umfangsrichtung umlaufenden Verstärkungsfasern 44 direkt versehen ist, so dass die Gehäusehaut 43 selbst das dehnfeste Element bildet. Oberhalb der horizontalen gestrichelten Linie ist radial innerhalb der Gehäusehaut 43 ein wendelförmiges Verstärkungselement 45 dargestellt, das durch eine Federstahlspirale gebildet sein kann. Die Spirale kann jedoch auch durch einen Kunststoff gebildet und in die Gehäusehaut eingegossen sein, wie unterhalb der gestrichelten Linie dargestellt.

In **Figur 12** ist ein Pumpengehäuse 39'' dargestellt, das aus einem Material besteht, das an sich selbst dehnfest ist und das Verstärkungselemente 46, 47, 48 umgibt, die jeweils einzeln als Kreisringe ausgebildet sind. Die einzelnen Kreisringe können zur Kompression des Gehäuses 39'' mit einer ausreichenden Kraft kollabiert oder derart gedreht werden, dass alle Kreisringe in der Zylinderachse des Gehäuses 39'' in derselben Ebene übereinanderliegen.

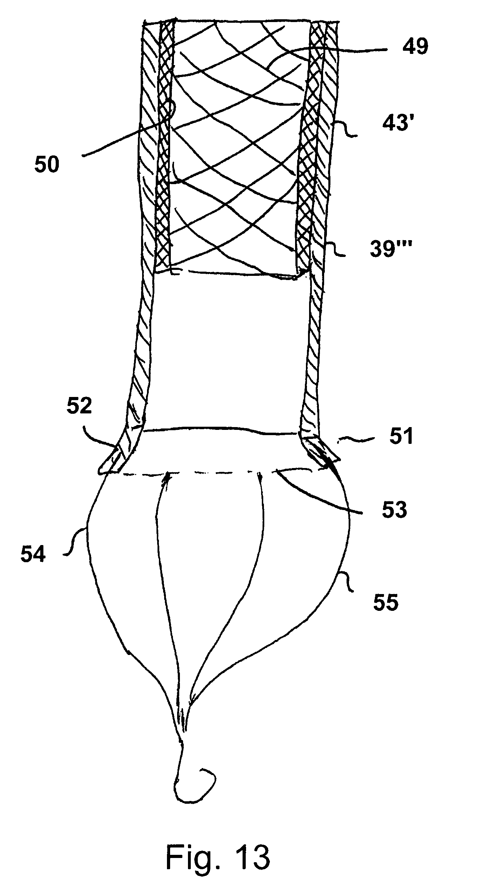

**Figur 13** zeigt eine Konstellation mit einem Pumpengehäuse 39''', wobei die Außenhaut 43' des Gehäuses aus einer dehnbaren Membran besteht und die radial innen liegenden, ein Geflecht bildenden Verstärkungsstreben 49, die auch im expandierten Zustand der Pumpe noch elastisch ein Stück weit komprimiert sind und auf eine weitere Expansion des Gehäuses hinwirken, durch ein dehnfestes Element 50 in Röhrenform gehalten sind. Das dehnfeste Element 50 ist zylindrisch geformt und in der Größe so bemessen, dass, wenn es durch die Verstärkungselemente 49 gespannt ist, auch die Gehäusehaut 43' entsprechend gespannt ist. Die elastischen Überschusskräfte der Verstärkungselemente 49, die als ein Drahtgeflecht ausgebildet sind, sind so bemessen, dass eine radial von außen auf das Gehäuse 39''' wirkende Kraft, sofern sie nicht 3 N übersteigt, nicht zu einer Verformung des Gehäuses 39"' und zu einer Reduzierung des Durchmessers des Gehäuseinnenraums führt.

An seinem distalen Ende 51 weist die Gehäusehaut 43' eine trichterförmige Erweiterung 52 auf, die das Einströmen von Blut durch die Ansaugöffnung 53 erleichtert. Gleichzeitig können in dem Fall, dass Verstärkungselemente in der Gehäusehaut 43' des Gehäuses 39''' verlaufen, diese aus der trichterförmigen Erweiterung 52 axial auslaufen und einen ballonartig erweiterten Ansaugkäfig bilden. Die in die Wand des Gehäuses 39''' integrierten beispielhaften Verstärkungselemente sind mit 54, 55 bezeichnet.

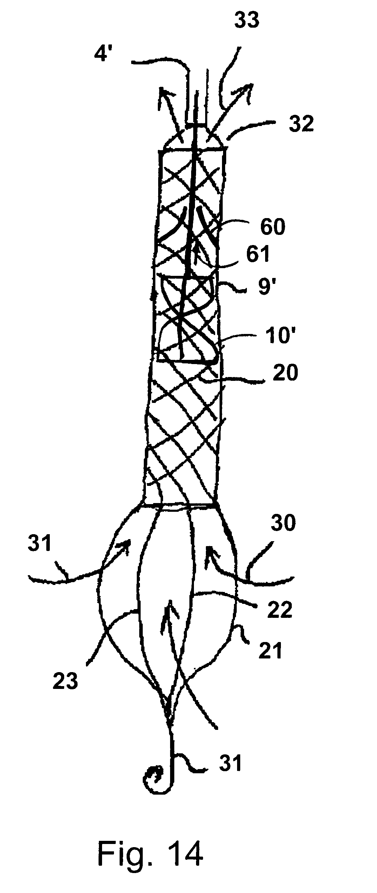

**Figur 14** zeigt in einer Seitenansicht analog Fig. 3 eine weitere Blutpumpe. Dargestellt ist darüber hinaus der Rotor 10' und proximal von diesem ein zusätzliches Ventil 60, welches das Zurückströmen des Blutes bei Stillstand der Pumpe verhindert, und auf diese Weise die Ventilfunktion ersetzt, die in Fig. 2 der dort dargestellt Abströmmantel 16 gemeinsam mit der Herzklappe 18 erfüllt. Das Ventil wird hier durch mehrere klappenartige, vorteilhaft folienartige Segel ausgebildet, welche sich unter dem Strömungsdruck der Pumpe in Richtung des Pfeils 61 öffnen und bei Stillstand der Pumpe wieder schließen. Die Segel können mit der Gehäusehaut einteilig ausgebildet oder an dieser befestigt sein. Bei der Komprimierung der Pumpe werden die Segel ebenfalls an die Wand des Gehäuses angelegt, um so die Durchmesserreduzierung auch in diesem Bereich zu gewährleisten.

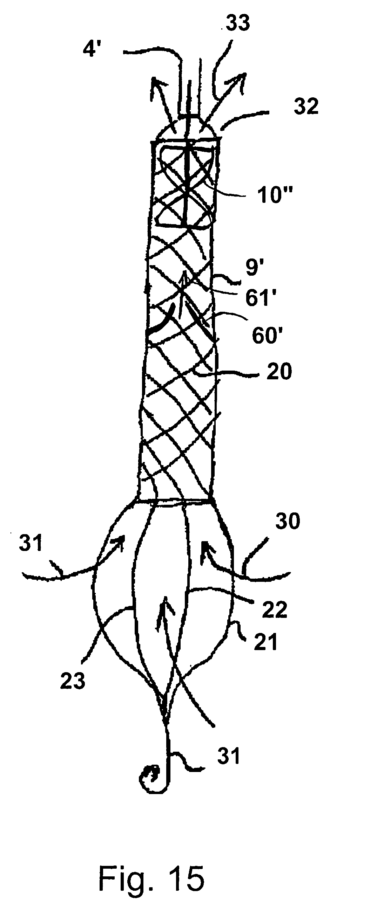

**Figur 15** zeigt eine Blutpumpe analog Fig. 14, wobei die Anordnung von Rotor und Ventil vertauscht ist. Diese Anordnung ist insofern vorteilhaft, dass die Antriebswelle des Rotors nicht innerhalb des Ventils verläuft.

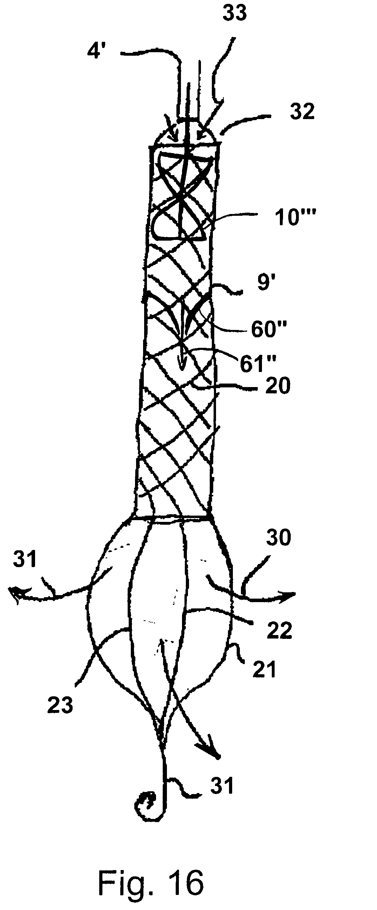

**Figur 16** zeigt eine Blutpumpe analog Fig. 15, wobei hier die Strömungsrichtung des Blutes umgekehrt ist. Derartige Pumpen, bei denen das Blut vom proximalen Ende des Gehäuses, an welchem der Katheter befestigt ist zum distalen Ende des Gehäuses strömt könne beispielsweise vorteilhaft zur Herzunterstützung im rechten Herzventrikel eingesetzt werden.

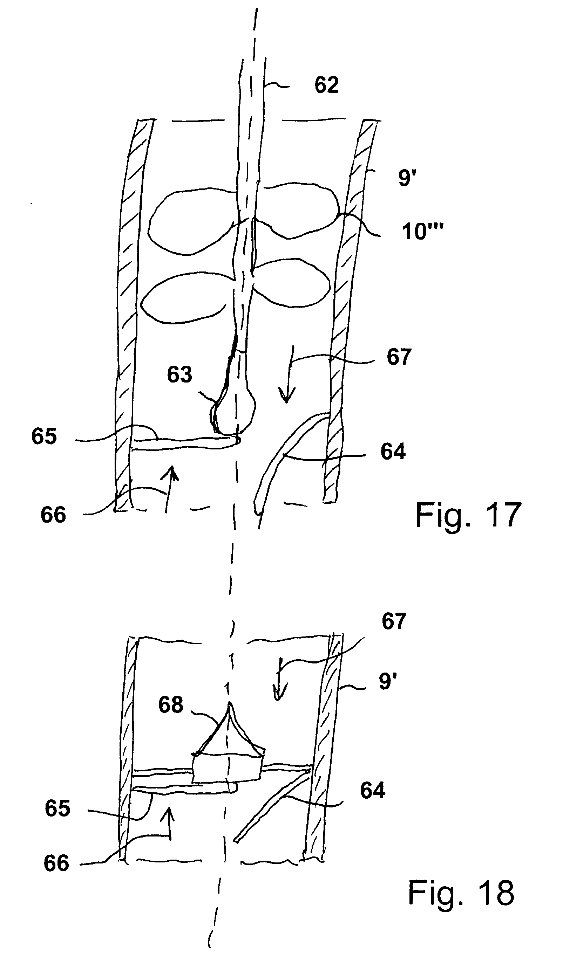

**Figur 17** zeigt im Längsschnitt, wie auch **Figur 18****,** ein Pumpengehäuse 9'. In Fig. 17 ist zudem ein Rotor 10''' dargestellt. In Verlängerung der Welle 62 ist ein Anschlagkörper 63 für die Segel 64, 65 eines Ventils gebildet, an dem die Segel im Falle einer Strömungsrichtung in Richtung des Pfeils 66 anschlagen. Bei einer Strömungsrichtung in Richtung des Pfeils 67 öffnen die Segel, die an der Gehäuseinnenwand befestigt sind, den Strömungskanal / das Ventil.

Fig. 17 zeigt einen Anschlagkörper, der sich in der Anströmrichtung verjüngt.

Fig. 18 zeigt einen Anschlagkörper, der in Anströmrichtung einen Konus 68 aufweist, jedoch auf seiner Abströmseite flach ausgebildet ist.

Die hier dargestellte Konstellation kann auch unabhängig von den Bedingungen des Hauptanspruchs allgemein bei Rotorpumpen, insbesondere komprimierbaren Rotorpumpen eingesetzt werden.

Weitere Aspekte betreffen unter anderem:
1. Blutpumpe mit einem Pumpengehäuse, das einen konisch zulaufenden Gehäuseinnenraum und einen in diesem angeordneten Rotor aufweist, wobei der Rotor in seiner Kontur zylindrisch gestaltet ist oder ebenfalls in seiner Außenkontur konisch in demselben Sinn zuläuft wie der Innenraum des Gehäuses und wobei die Konuswinkel der Außenkontur und des Gehäuseinnenraums annähernd gleich sind.
2. Verfahren zur Justierung einer Blutpumpe gemäß dem ersten Aspekt, wobei der Rotor im Rotationsbetrieb gegenüber dem Pumpengehäuse axial so lange verschoben wird, bis durch Ermittlung der Last des Antriebsmotors der Pumpe sichergestellt ist, dass der Rotor sich im Pumpengehäuse berührungsfrei dreht.
3. Pumpengehäuse (9, 9', 9'', 9''', 9'''', 39, 39', 39'', 39''') mit einem Innenraum zur Aufnahme eines Pumpenrotors (10, 10', 10'', 10'''), welches aus einem radial komprimierten Zustand in einen radial expandierten Zustand überführbar ist wobei axial beanstandet zum Rotor wenigstens ein Element zur Änderung des Strömungswiderstandes für eine durch den Innenraum hindurchgehende Fluidströmung, insbesondere ein Ventil, vorgesehen ist.
4. Pumpengehäuse gemäß Aspekt 1, wobei das Element, insbesondere das Ventil, im Innenraum des Gehäuses ausgebildet ist.
5. Pumpengehäuse gemäß einem der vorstehenden Aspekte, wobei das wenigstens eine Element, insbesondere das Ventil, aus einem radial komprimierten Zustand in einen radial expandierten Zustand überführbar ist.
6. Pumpengehäuse gemäß einem der vorstehenden Aspekte, wobei die Ventilfunktion im expandierten Zustand ausgeübt wird.
7. Pumpengehäuse gemäß einem der vorstehenden Aspekte, wobei das Element wenigstens ein schwenkbares und/oder verformbares Flächenelement, insbesondere in Form eines Segels oder einer Klappe, aufweist, das unter Strömungsdruck in einer ersten Strömunsrichtung einen Strömungskanal öffnet und bei Stillstand der Pumpe bzw. bei entgegengerichtetem Strömungsdruck schließt.
8. Pumpengehäuse gemäß einem der vorstehenden Aspekte, wobei das Element / die Elemente als folienartiges Segel ausgebildet ist/sind.
9. Pumpengehäuse gemäß einem der vorstehenden Aspekte, wobei wenigstens eines der Elemente am Gehäuse befestigt ist.
10. Pumpengehäuse gemäß einem der vorstehenden Aspekte, wobei das Element / die Elemente einteilig mit der Gehäusehaut ausgebildet ist/sind.
11. Pumpengehäuse gemäß einem der vorstehenden Aspekte, wobei wenigstens eines der Elemente an die Innenseite der Gehäusewand anlegbar und von dieser weg schwenkbar ist.
12. Pumpengehäuse gemäß einem der vorstehenden Aspekte, wobei im Inneren des Gehäuses ein fester Körper befestigt ist, an dem das/die Elemente im geschlossenen Zustand des Ventils anschlagen.

## Patentansprüche

1. Pumpengehäuse (9, 9', 9'', 9''', 9'''', 39, 39', 39'', 39''') mit einem Innenraum zur Aufnahme eines Pumpenrotors (10, 10', 10'', 10'''), welches aus einem radial komprimierten Zustand in einen radial expandierten Zustand überführbar ist und eine in seiner Umfangsrichtung umlaufende Gehäusehaut (43, 43') sowie wenigstens ein Verstärkungselement (20, 21, 22, 23, 24, 25, 26, 27, 28, 29, 45, 46, 47, 48, 49) aufweist, wobei im voll expandierten Zustand des Gehäuses die Gehäusehaut in Umfangsrichtung durch die Expansion des Verstärkungselementes / der Verstärkungselemente gespannt ist und wobei wenigstens ein in Umfangsrichtung umlaufendes dehnfestes Element (37, 43, 43', 50) vorgesehen ist, das im expandierten Zustand gegenüber dem kraftfreien Zustand in Umfangsrichtung weniger als 5% gedehnt ist und das eine weitere Expansion des Pumpengehäuses begrenzt.

2. Pumpengehäuse gemäß Anspruch 1, **dadurch gekennzeichnet, dass** die elastischen, auf eine weitere radiale Expansion des Gehäuses gerichteten Kräfte des Verstärkungselements / der Verstärkungselemente (20, 21, 22, 23, 24, 25, 26, 27, 28, 29, 45, 46, 47, 48, 49) im expandierten Zustand des Gehäuses so groß sind, dass die radial nach außen gerichteten Kräfte größer sind als 1 N, vorteilhaft mindestens 4 N.

3. Pumpengehäuse gemäß Anspruch 1, **dadurch gekennzeichnet, dass** ein durch das Verstärkungselement / die Verstärkungselemente (20, 21, 22, 23, 24, 25, 26, 27, 28, 29, 45, 46, 47, 48, 49) im expandierten Zustand des Gehäuses gebildetes Gerüst oder Gewölbe radial nach innen gerichteten Kräften von mindestens 1 N, vorteilhaft mindestens 4 N widersteht, ohne einzufallen.

4. Pumpengehäuse gemäß Anspruch 1 oder einem der folgenden, **dadurch gekennzeichnet, dass** das dehnfeste Element (37, 43, 43', 50) durch eine biegeschlaffe Folie, insbesondere durch die Gehäusehaut (43, 43'), gebildet ist.

5. Pumpengehäuse gemäß Anspruch 1, **dadurch gekennzeichnet, dass** das dehnfeste Element (37, 43, 43', 50) durch einen in Umfangsrichtung des Gehäuses umlaufenden, wenigstens abschnittsweise das Verstärkungselement umgebenden Ring (37, 50) gebildet ist.

6. Pumpengehäuse gemäß Anspruch 5, **dadurch gekennzeichnet, dass** das dehnfeste Element (37) an der radial äußeren Seite der Gehäusehaut (43, 43') angeordnet ist.

7. Pumpengehäuse gemäß Anspruch 5, **dadurch gekennzeichnet, dass** das dehnfeste Element (37) an der radial inneren Seite der Gehäusehaut (43, 43') angeordnet ist.

8. Pumpengehäuse gemäß Anspruch 4 oder 5, **dadurch gekennzeichnet, dass** das dehnfeste Element (43) in Umfangsrichtung verlaufende dehnfeste Fasern (44), insbesondere Glasfasern, Kohlenstofffasern und/oder Aramidfasern und/oder Nylonfasern aufweist.

9. Pumpengehäuse gemäß Anspruch 1 oder einem der folgenden, **dadurch gekennzeichnet, dass** die Verstärkungselemente ein flächenartiges, zweidimensionales Gitter (20, 49) bilden, das in die Form einer Röhre gebogen ist.

10. Pumpengehäuse gemäß Anspruch 1 oder einem der folgenden, **dadurch gekennzeichnet, dass** mehrere gegeneinander schwenkbare Verstärkungselemente (27, 28) in einem ersten Schwenkzustand gemeinsam die Form einer Röhre bilden und in einem zweiten Schwenkzustand radial gegenüber der Röhrenform komprimiert sind.

11. Pumpengehäuse gemäß Anspruch 1 oder einem der folgenden, **dadurch gekennzeichnet, dass** die den Gehäuseinnenraum begrenzende Innenwand (56) in Axialrichtung sich verjüngend, insbesondere konisch, zuläuft.

12. Pumpengehäuse gemäß Anspruch 1 oder einem der folgenden, **dadurch gekennzeichnet, dass** das Verstärkungselement / die Verstärkungselemente (20, 21, 22, 23, 24, 25, 26, 27, 28, 29, 45, 46, 47, 48, 49) aus einem superelastischen Werkstoff, insbesondere einer superelastischen Legierung, weiter insbesondere Nitinol, besteht/bestehen.

13. Blutpumpe mit einem Pumpengehäuse (9, 9', 9'', 9''', 9'''', 39, 39', 39'', 39''') gemäß Anspruch 1 oder einem der folgenden und mit einem Rotor (10, 10', 10'', 10''').

14. Blutpumpeneinrichtung mit einer Blutpumpe mit einem Pumpengehäuse gemäß Anspruch 11 und mit einem an die Blutpumpe anschließenden Katheter (4, 4').

15. Blutpumpe nach Anspruch 13, **dadurch gekennzeichnet, dass** der Rotor radial komprimierbar und expandierbar ausgestaltet ist, und wobei der Rotor in wenigstens einem Betriebszustand im Längsschnitt bezüglich des bei der Rotation beanspruchten Raumes eine konkave und/oder konvexe Außenkontur aufweist und wobei der Gehäuseinnenraum im Längsschnitt eine an die Außenkontur des Rotors angepasste Kontur aufweist.

## Claims

1. A pump housing (9, 9', 9", 9'", 9'''' 39, 39', 39", 39'") having an interior for accommodating a pump rotor (10, 10', 10", 10''), which may be transferred from a radially compressed state into a radially expanded state, and comprises a housing skin (43, 43') running circumferentially around it, as well as at least one reinforcement element (20, 21, 22, 23, 24, 25, 26, 27, 28, 29, 45, 46, 47, 48, 49), wherein in the fully expanded state of the housing the housing skin is stretched in circumferential direction by means of the expansion of the reinforcement element(s), and wherein at least one stretch-resistant element (37, 43, 43', 50) running in circumferential direction is provided, which is stretched in the expanded state as opposed to the force-free state in circumferential direction less than 5%, and which limits any further expansion of the pump housing.

2. The pump housing according to claim 1, **characterized in that** the elastic forces of the reinforcement element(s) (20, 21, 22, 23, 24, 25, 26, 27, 28, 29, 45, 46, 47, 48, 49) directed towards an additional radial expansion of the housing, are so great in the expanded state of the housing that the forces directed radially toward the exterior are greater than 1 N, advantageously, at least 4 N.

3. The pump housing according to claim 1, **characterized in that** a vault or an arch formed by means of the reinforcement element(s) (20, 21, 22, 23, 24, 25, 26, 27, 28, 29, 45, 46, 47, 48, 49) in the expanded state of the housing resists forces directed radially toward the interior of at least 1 N, advantageously of at least 4 N, without collapsing.

4. The pump housing according to claim 1, or one of the following, **characterized in that** the stretch-resistant element (37, 43, 43', 50) is formed by means of a flexible film, in particular by means of the housing skin (43, 43').

5. The pump housing according to claim 1, **characterized in that** the stretch-resistant element (37, 43, 43', 50) is formed by means of a ring (37, 50) running in circumferential direction of the housing, surrounding the reinforcement element at least in sections.

6. The pump housing according to claim 5, **characterized in that** the stretch-resistant element (37) is disposed on the radially external side of the housing skin (43, 43').

7. The pump housing according to claim 5, **characterized in that** the stretch-resistant element (37) is disposed on the radially internal side of the housing skin (43, 43').

8. The pump housing according to claim 4 or claim 5, **characterized in that** the stretch-resistant element (43) comprises stretch-resistant fibers (44) extending in circumferential direction, in particular glass fibers, carbon fibers, and/or aramide fibers, and/or nylon fibers.

9. The pump housing according to claim 1, or one of the following, **characterized in that** the reinforcement elements form a surface-like, two-dimensional grate (20, 49), which is bent into the form of a tube.

10. The pump housing according to claim 1, or one of the following, **characterized in that** multiple reinforcement elements (27, 28) that are pivotable against each other form the shape of a tube in a first pivoting state, and are compressed radially as opposed to the tube shape in a second pivoting state.

11. The pump housing according to claim 1, or one of the following, **characterized in that** the interior wall (56) limiting the housing interior tapers in axial direction, in particular in a conical manner.

12. The pump housing according to claim 1, or one of the following, **characterized in that** the reinforcement element(s) (20, 21, 22, 23, 24, 25, 26, 27, 28, 29, 45, 46, 47, 48, 49) are comprised of a super-elastic material, in particular a super-elastic alloy, and in particular nitinol.

13. A blood pump having a pump housing (9, 9', 9", 9"', 9"", 39, 39', 39", 39"') according to claim 1, or one of the following, and having a rotor (10, 10', 10", 10"').

14. A blood pump unit having a blood pump with a pump housing according to claim 11, and having a catheter (4, 4') attached to the blood pump.

15. The blood pump of claim 13, **characterized in that** the rotor is configured to be radially compressible and expandable, and wherein the rotor has a concave and/or convex exterior contour in at least one operating state at a longitudinal section with regard to the space occupied during rotation, and wherein the housing interior has a contour in a longitudinal section that is adapted to the exterior contour of the rotor.

## Revendications

1. Boîtier de pompe (9, 9', 9'', 9''', 9'''', 39, 39', 39'', 39''') avec un espace intérieur pour la réception d'un rotor de pompe (10, 10', 10'', 10'''),
lequel peut être transféré d'un état comprimé radialement à un état expansé radialement et présente une enveloppe de boîtier (43, 43') parcourant sa direction circonférentielle ainsi qu'au moins un élément de renforcement (20, 21, 22, 23, 24, 25, 26, 27, 28, 29, 45, 46, 47, 48, 49), où, dans l'état totalement expansé du boîtier, l'enveloppe de boîtier est sous tension dans la direction circonférentielle par l'expansion de l'élément de renforcement / des éléments de renforcement et où au moins un élément résistant à la traction (37, 43, 43', 50) parcourant la direction circonférentielle est prévu, qui, dans l'état expansé, est étiré à moins de 5 % dans la direction circonférentielle par rapport à l'état non sollicité et qui limite une expansion ultérieure du boîtier de pompe.

2. Boîtier de pompe selon la revendication 1, **caractérisé en ce que** les forces de l'élément de renforcement / des éléments de renforcement (20, 21, 22, 23, 24, 25, 26, 27, 28, 29, 45, 46, 47, 48, 49) élastiques, orientées envers une nouvelle expansion radiale du boitier à l'état expansé du boîtier sont si importantes que les forces dirigées radialement vers l'extérieur sont supérieures à 1 N, de manière avantageuse, à au moins 4 N.

3. Boîtier de pompe selon la revendication 1, **caractérisé en ce qu'**une ossature, ou une voûte, formée par l'élément de renforcement / les éléments de renforcement (20, 21, 22, 23, 24, 25, 26, 27, 28, 29, 45, 46, 47, 48, 49) à l'état expansé du boîtier résiste à des forces orientées radialement vers l'intérieur d'au moins 1 N, de manière avantageuse, d'au moins 4 N, sans s'écrouler.

4. Boîtier de pompe selon la revendication 1 ou l'une des suivantes, **caractérisé en ce que** l'élément résistant à la traction (37, 43, 43', 50) est formé par un film relâché de manière flexible, notamment par l'enveloppe de boîtier (43, 43').

5. Boîtier de pompe selon la revendication 1, **caractérisé en ce que** l'élément résistant à la traction (37, 43, 43', 50) est formé par une bague (37, 50) parcourant le boîtier dans la direction circonférentielle, entourant au moins par endroits l'élément de renforcement.

6. Boîtier de pompe selon la revendication 5, **caractérisé en ce que** l'élément résistant à la traction (37) est disposé sur la face extérieure radiale de l'enveloppe de boîtier (43, 43').

7. Boîtier de pompe selon la revendication 5, **caractérisé en ce que** l'élément résistant à la traction (37) est disposé sur la face intérieure radiale de l'enveloppe de boîtier (43, 43').

8. Boîtier de pompe selon les revendications 4 ou 5, **caractérisé en ce que** l'élément résistant à la traction (43) présente des fibres résistantes à la traction (44) parcourant la direction circonférentielle, notamment des fibres de verre, des fibres de carbone et/ou des fibres d'aramide et/ou des fibres de Nylon.

9. Boîtier de pompe selon la revendication 1 ou l'une des suivantes, **caractérisé en ce que** les éléments de renforcement forment un réseau (20, 49) à deux dimensions, de forme plane, qui est coudé sous la forme d'un tube.

10. Boîtier de pompe selon la revendication 1 ou l'une des suivantes, **caractérisé en ce que** plusieurs éléments de renforcement (27, 28) pouvant pivoter les uns vers les autres dans un premier état pivoté forment ensemble la forme d'un tube et sont comprimés radialement par rapport à la forme du tube dans un deuxième état pivoté.

11. Boîtier de pompe selon la revendication 1 ou l'une des suivantes, **caractérisé en ce que** la paroi intérieure (56) délimitant l'espace intérieur de boîtier dans la direction axiale, s'étend dans la direction axiale en s'amenuisant, notamment de manière conique.

12. Boîtier de pompe selon la revendication 1 ou l'une des suivantes, **caractérisé en ce que** l'élément de renforcement / les éléments de renforcement (20, 21, 22, 23, 24, 25, 26, 27, 28, 29, 45, 46, 47, 48, 49) est constitué / sont constitués d'un matériau super élastique, notamment d'un alliage super élastique, notamment encore de Nitinol.

13. Pompe à sang avec un boîtier de pompe (9, 9', 9'', 9''', 9'''', 39, 39', 39'', 39''') selon la revendication 1 ou l'une des suivantes, et avec un rotor (10, 10', 10'', 10''').

14. Dispositif de pompe à sang avec une pompe à sang, avec un boîtier de pompe selon la revendication 11 et avec un cathéter (4, 4') relié à la pompe à sang.

15. Pompe à sang selon la revendication 13, **caractérisée en ce que** le rotor est conçu en pouvant être comprimé radialement et en pouvant être expansé, et où le rotor présente dans au moins un état de fonctionnement, dans la coupe longitudinale par rapport à l'espace sollicité lors de la rotation, un contour extérieur concave et/ou convexe et où l'espace intérieur de boîtier présente dans la coupe longitudinale un contour ajusté au contour extérieur du rotor.
